# EUROPEAN PATENT APPLICATION

(11) **EP 4 685 134 A1**
(43) Date of publication of application: **28.01.2026**
(21) Application number: 24191183.3
(22) Date of filing: 26.07.2024
(51) Int. Cl.: C07C 209/84, C07C 211/12

(54) **METHOD FOR SEPARATING HEXAMETHYLENE DIAMINE FROM AN AQUEOUS SOLUTION OBTAINED FROM A MICROBIAL FERMENTATION PROCESS**

(71) Applicant: Covestro AG, 51373 Leverkusen (DE); Genomatica, Inc., San Diego CA 92121 (US)
(72) Inventor: Leimbrink, Mathias, 51373 Leverkusen (DE); Krause, Jonas, 51373 Leverkusen (DE); Altuntepe, Emrah, 51373 Leverkusen (DE); Bausa, Jürgen, 51373 Leverkusen (DE); Waltermann, Thomas, 51373 Leverkusen (DE); Galleher, Connor, San Diego, CA 92121 (US); Chen, Bo, San Diego, CA 92121 (US); Safari, Iman, San Diego, CA 92121 (US)
(74) Representative: Scholz, Volker

(57) **Abstract**

The present invention provides a method for separating hexamethylene diamine (HMDA) from an aqueous solution, comprising the steps: a) providing an HMDA-containing aqueous solution, wherein said aqueous solution is a fermentation broth, b) extracting the HMDA-containing aqueous solution with a monovalent alcohol having 4 to 8 carbon atoms, c) obtaining an HMDA-containing alcoholic phase, and d) distilling the HMDA-containing alcoholic phase at a pressure of 1-1000 mbar and separating the HMDA from the monovalent alcohol.

## Description

The present invention relates to a method for separating hexamethylene diamine from an aqueous solution originating from a microbial fermentation process.

Hexamethylene diamine (HDA, HMDA, or hexane-1,6-diamine) , is an important starting material in the production of polymers. It is used, for example, for the production of polyamides by polycondensing the diamine with dicarboxylic acids. Polyamide-6.6 (PA66), for example, is generated from hexamethylene diamine and adipic acid. Another important use is the phosgenation of HMDA to the corresponding diisocyanate, which can then be converted in further steps into polyurethanes, polyureas or polyisocyanurates.

Industrial production methods have become established for some years in which HMDA is produced by biotechnological means. Here, HMDA is first of all generated as a mixture with water, and the mixture is then processed into pure diamine. Recovering a diamine from the aqueous fermentation medium can be carried out as disclosed in EP 2684867 A1, for example, after most of the cell components have been removed. There, a method for the production of pentamethylene diamine is disclosed, in which the diamine is extracted in a liquid phase extraction process from the aqueous phase that has optionally been concentrated by distillation beforehand. As extractants, non-halogen aliphatic solvents, preferably straight chain alcohols with 4 to 7 carbon atoms, are recommended. These extractants must subsequently be separated from the product again by distillation. For the distillation processes to concentrate the aqueous raw product or to isolate the pentamethylene diamine from the extract, the use of one or more distillation columns and a pressure of between 0.1 kPa and normal pressure is described in each case.

Biotechnological production processes are also known for the production of hexamethylene diamine. For example, US 20170369913 A1 discloses extraction with various extractants and distillation of hexamethylene diamine (which has been produced by biological means).

There is still a need for processes for separating HMDA from an aqueous solution originating from a microbial fermentation process having improved energy efficiency.

It is therefore the object of the present invention to provide a process for the separation of HMDA from an aqueous solution originating from a microbial fermentation process having improved energy efficiency.

This object is solved by a method for separating hexamethylene diamine (HMDA) from an aqueous solution, comprising the steps:
a) providing an HMDA-containing aqueous solution, wherein said aqueous solution is a fermentation broth,
b) extracting the HMDA-containing aqueous solution with a monovalent alcohol having 4 to 8 carbon atoms,
c) obtaining an HMDA-containing alcoholic phase, and
d) distilling the HMDA-containing alcoholic phase at a pressure of 1-1000 mbar and separating the HMDA from the monovalent alcohol.

The HMDA-containing aqueous solution is a fermentation broth. A "fermentation broth" as referred to in the present application is an aqueous composition which originates from a microbial fermentation process. This microbial fermentation process is, preferably, a process, wherein microorganisms, preferably bacteria, are provided with one or more organic compounds that are converted to HMDA by the metabolic activity of said microorganisms. As microbial cells release further products in addition to HMDA into the fermentation broth and a suitable fermentation broth must comprise a range of nutrients such as amino acids or vitamins in order to support the activity of the microorganisms, it is clear that the aqueous solution must comprise organic compounds in addition to HMDA.

Therefore, in a preferred embodiment of the present invention, said aqueous solution additionally comprises at least one compound selected from the group consisting of (i) acetate, (ii) lactate, (iii) glutamate, (iv) ammonium and (v) γ-aminobutyric acid.

The compounds recited above are typical by-products of microbial fermentations. Acetate is a short-chain fatty acid which originates from a multitude of aerobic as well anaerobic metabolic pathways in microorganisms, e.g. butyric acid fermentation or by cleavage of acetyl-coenzyme A. Lactate is a product of glycolysis. Glutamate is a proteinogenic amino acid. Ammonium is found in many fermentation broths as source of nitrogen. It may also be released during amino acid metabolism, γ-aminobutyric acid is produced by a multitude of microorganisms such as certain *Lactobacilli* or yeasts.

Acetate is preferably present in a concentration range between 1 and 4,000 mg/L, more preferably 200 and 3,000 mg/L and most preferably 750 to 2,500 mg/L.

Lactate is preferably present in a concentration range between 1 and 400 mg/L, more preferably 5 and 200 mg/L and most preferably 10 to 100 mg/L.

Glutamate is preferably present in a concentration range between 10 and 1,000 mg/L, more preferably 50 and 600 mg/L and most preferably 75 to 250 mg/L.

Ammonium is preferably present in a concentration range between 1 and 200 mg/L, more preferably 5 and 120 mg/L and most preferably 10 to 80 mg/L.

y-aminobutyric acid is preferably present in a concentration range between 300 and 4,000 mg/L, more preferably 500 and 3,000 mg/L and most preferably 700 to 2,000 mg/L.

The present application does not relate to the separation of HMDA from an aqueous solution, if the HMDA originates from the depolymerization of a polymer. Such an aqueous solution is not the product of a microbial fermentation process but from the chemical process used for the depolymerization.

The HMDA is preferably present in uncharged form. This is typically the case at a pH of at least 11.5. Therefore, the HMDA-containing aqueous solution in method step a) preferably has a pH of at least 11.5, more preferably 12.0..

The monovalent alcohol may be selected from the group consisting of 1-pentanol, 1-hexanol, 1-heptanol or mixtures thereof.

The HMDA-containing alcoholic phase may be distilled in step d) at a pressure of 50-800 mbar, 100-600 mbar, or 150-400 mbar.

Further, the HMDA-containing alcoholic phase may be distilled in step d) with a single distillation column.

Moreover, step b) may be carried out using an extraction column and/or a mixer-settler, having 3 to 15 theoretical stages.

In accordance with the invention, the expressions "comprising", "including" or "containing" mean preferably "consisting substantially of" and particularly preferably "consisting of".

Statements concerning the content of organic compounds in the context of the present invention refer, unless stated otherwise, to values determined by gas chromatography. The skilled person is familiar with the quantitative evaluation of gas chromatograms, optionally using an internal standard.

"Extraction" in the present case is understood to mean a separation process consisting of the separation of a substance from a matrix, wherein in the present case substance and matrix are both liquids to result in a liquid-liquid extraction. Two immiscible phases are combined to separate a solute from one phase into the other, according to the relative solubility in each of the phases. Suitable extraction devices for the liquid-liquid extraction are well known in the art.

"Distillation" in the present case is understood to mean a thermal separation process used to recover evaporable substances, preferably liquids, from a composition. These separated vapors are subsequently precipitated, usually by condensation. The term encompasses in particular repeated evaporation and condensation using a column (distillation column) with a plurality of separating stages. Processes of this kind in which several distillation steps are arranged in a series in a column should strictly speaking be called rectification, but will nevertheless likewise be referred to herein as distillation for the purpose of simplification. The advantage of these processes is the powerful separating effect and the possibility of operating the plant continuously. A "distillation apparatus" is accordingly an apparatus which is suitable for performing a thermal separation process of this kind, i.e. for example sieve plate columns, packed columns, packed-bed columns, bubble cap tray columns or even single-stage evaporators such as, for example, falling film evaporators, thin-film evaporators, flash evaporators, multiphase helical-coil evaporators, or natural or forced circulation evaporators.

The individual process steps will be explained in more detail below.

In the first step a) of the method of the invention, an HMDA-containing aqueous solution is provided. The weight ratio of HMDA and water can be freely chosen, e.g., the HMDA can be present at a maximum amount of about 50 wt.-%, based on the total weight of water and HMDA, to allow efficient extraction of the HMDA from a respective concentrated HMDA-containing aqueous solution. The skilled person may choose a minimum amount of the HMDA of about 0.1 wt.-%, based on the total weight of water and HMDA, and may choose a maximum amount of the diamine depending on the HMDA and temperature used as long as a two phase system may still be achieved when mixed with the monovalent alcohol extractant.

Preferably, the source of the HMDA to be separated according to the method of the present invention is restricted to HMDA prepared by biotechnological means such as fermentation, enzymatic catalysis, or whole cell bio-catalysis. However, it is not HMDA obtained from recycling of polyamides or polyurethanes even in cases, where the HMDA used in the synthesis of said polymers was originally obtained by the biotechnological means defined above in this paragraph.

In the second step b) of the method of the invention, the HMDA-containing aqueous solution provided in step a) is extracted with a monovalent alcohol having 4 to 8 carbon atoms.

The monovalent alcohol must enable an efficient extraction of the HMDA. If too much water is carried over, the energy consumption of the distillation increases. Further, the monovalent alcohol must also be able to carry a high concentration of HMDA, so that the amount of alcohol required for the extraction of the HMDA is minimized as distillation of the alcohol requires energy.

The monovalent alcohol is not particularly restricted as long as it has 4 to 8 carbon atoms, and the monovalent alcohol may include all possible isomers. The monovalent alcohol may be selected from 1-butanol, i-butanol, sec-butanol, 1-pentanol, 2-pentanol, 3-pentanol, neopentyl alcohol, 1-hexanol, 2-hexanol, 3-hexanol, 1-heptanol, 2-heptanol, 3-heptanol, 4-heptanol, 1-octanol, 2-octanol, 3-octanol, 4-octanol, cyclopentanol, cyclohexanol, cycloheptanol or cyclooctanol or mixtures thereof. In a preferred embodiment of the present invention the monovalent alcohol may be selected from 1-pentanol, 1-hexanol, 1-heptanol or mixtures thereof. In a more preferred embodiment of the present invention the monovalent alcohol may be 1-hexanol.

Step b) may be carried out by any suitable extracting device, which is known in the art. Extraction may be carried out using an extraction column and/or a mixture-settler, having 3 to 15 theoretical stages.

Extraction conditions are not limited, the extraction may be carried out at room temperature (20-25°C) and/or under ambient pressure. The higher the temperature the higher the partition coefficient, thus extraction efficiency is increased. The extraction temperature is limited by the boiling temperature of water or the monovalent alcohol used as extractant. The extraction may be carried out under ambient pressure. A phase ratio, i.e., mass ratio, of alcohol:water can be from 0.1 to 1, 0.2 to 0.5 or 0.3 to 0.4.

In the third step c) of the method of the invention, an HMDA-containing alcoholic phase is obtained. This HMDA-containing alcoholic phase is the organic phase obtained after extraction in step b). The other phase is the aqueous phase. As HMDA has a higher solubility in the monovalent alcohol than in water, the HMDA will be concentrated in the organic alcoholic phase. The HMDA-containing alcoholic phase may contain, besides HMDA and monovalent alcohol, also residual amounts of water.

The HMDA-containing alcoholic phase (organic phase) may be easily separated from the aqueous phase after extraction by means well known in the art.

In the fourth step d) of the method of the invention, the HMDA-containing alcoholic phase obtained in step c) is distilled at a pressure of 1-1,000 mbar, and, thus, the HMDA is separated from the monovalent alcohol and the optionally residual amounts of water.

It was surprisingly found that the efficiency of the distillation step d) depends on the applied pressure as the equilibrium of vapor and liquid phases is highly pressure-dependent.

Best distillation results are achieved within a pressure optimum for the distillation step.

A distillation device used in step d) is not limited, but any distillation device known in the art for such separation steps may be used. The distillation device may be a single distillation column. The single distillation column may have 5 to 100, 10 to 60 or 20 to 50 theoretical stages. In case, 1-hexanol is the monovalent alcohol, 20 or more theoretical stages, e.g., 20 to 50 theoretical stages, may be used. The distillation pressure may be from 100-1,000 mbar, 150-800 mbar, 150-700 mbar, 200-600 mbar, 200-500 mbar or 200-400 mbar. It is, thus, lower than the atmospheric pressure of 1,013 mbar.

The distillation pressure may be 200-400 mbar, the monovalent alcohol may be 1-hexanol. The distillation pressure may be 200-400 mbar, the monovalent alcohol may be i-butanol

Typically, during the distillation, the monovalent alcohol used has a lower boiling point than the HMDA and is evaporated to the top of the distillation device, may be condensed and may then be removed from the distillation device and may be optionally reused as extractant. HMDA typically has the higher boiling point and is, thus, concentrated at the bottom of the distillation device and may be removed therefrom for further processing.

The HMDA-containing aqueous solution may contain substances having higher or lower boiling points compared to HMDA.

Substances with lower boiling points compared to HMDA are, for example, apart from water, ammonia, etc. In addition, gases such as nitrogen or carbon dioxide may be present in the composition in dissolved or bound form.

Substances with higher boiling points compared to HMDA can occur. Preferably, these are carbohydrates which have not been completely converted during the fermentation process and salts that originate from the fermentation broth and are now still present may be contained.

### Brief description of the drawings

Fig. 1 is a graph illustrating the dependency of the ideal separation factor on temperature for a mixture of C6-diamine (HMDA) and different monovalent alcohols;
Fig. 2 is a graph illustrating the dependency of the ideal separation factor on the temperature for a mixture of C6-monoamine (hexaneamine) and different monovalent alcohols;
Fig. 3 is a graph illustrating the dependency of the ideal separation factor on the temperature for a mixture of a C6-diamine (HMDA) and different divalent alcohols;
Fig. 4 is a graph illustrating the dependency of the ideal separation factor on the temperature for a mixture of a C6-monoamine (hexaneamine) and different divalent alcohols;
Fig. 5 is a graph illustrating vapor-liquid equilibrium (VLE) data for a mixture of 1-hexanol and hexamethylene diamine; experimental data (data points) was generated with a circulating still - an isobaric dynamic method (here only data at 1000 mbar is shown). The ideal VLE of 1-hexanol and hexamethylene diamine was calculated and an UNIFAC estimation was performed, as well. Experimental data was used to regress NRTL parameters. The calculation with NRTL using these parameters is also shown.
Fig. 6 is a graph illustrating the effect of operating pressure on energy demand in a distillation step of the present invention. It is the distillation of HMDA/1-hexanol. The temperature during distillation is dependent on the pressure and is not constant. The heat duty is the totally required heat input. The graph shows a calculated example using the software Aspentech Aspen plus based on parameters derived from the illustrated measurements.

### Examples

The present invention is further illustrated by means of examples and comparative examples. The examples shall not restrict the scope of protection which is solely defined by the claims.

In the inventive method, step d) of distilling the HMDA-containing alcoholic phase is the most essential step. To illustrate that advantageous effects may be achieved when distilling an appropriately selected combination of diamine and monovalent alcohol, several combinations of monovalent and divalent alcohols with mono- and diamines, respectively, were investigated. All vapor pressures of pure components are reported in literature and available data was used.

Four different types of combinations were investigated, as follows:
1. monovalent alcohol + diamine
2. monovalent alcohol + monovalent amine
3. divalent alcohol + diamine
4. divalent alcohol + monovalent amine

The combination of monovalent alcohol + diamine is according to the invention, the other combinations are comparative examples.

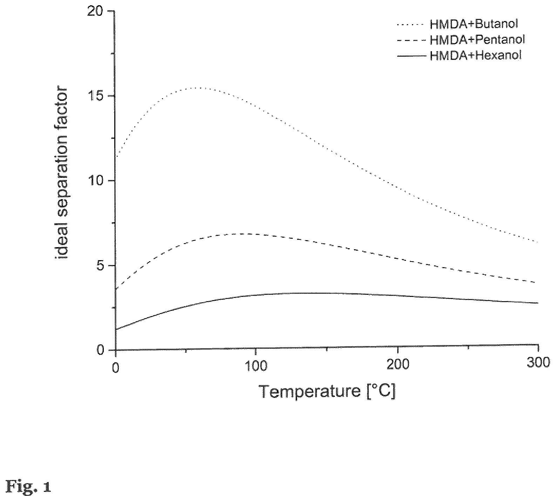

In the examples, hexanediamine was used as diamine and hexanamine was used as monoamine. The same findings as shown in Fig. 1-4 account also for C4 amines and diamines. C4 to C6 monovalent and divalent alcohols are investigated in these studies as given in Fig. 1-4.

(Ideal) relative volatilityFor each example and comparative example of Fig. 1-4, a mixture of amine and alcohol was used to calculate, based on literature values, the (ideal) relative volatility (also referred to as separation factor) which is the ratio of the vapor pressures of the pure compounds of amine and alcohol at a given temperature, where the vapor pressure of the low-boiler is divided by the vapor pressure of the high-boiler.

The separation factors for the respective mixtures are summarized in Fig. 1-4.

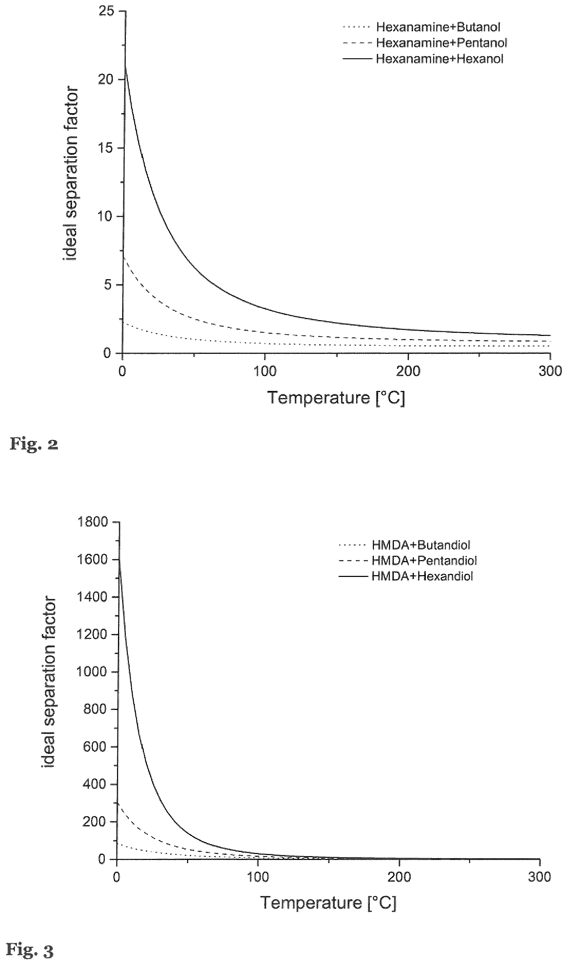

From Fig. 1-4 it can be seen that the combinations of monovalent alcohols and diamines appear to behave in a totally unexpected manner. From basic textbook knowledge and also from the behavior of similar combinations (Fig. 2 to 4) it is always assumed that a separation gets easier with lowering the pressure (separation factor increases steadily with lowering the pressure - corresponds to lowering the temperature). But the separation factor of mixtures of monovalent alcohols and hexanediamine shows a specific maximum (this also accounts for putrescine in mixtures with C4 to C6 monovalent alcohols).

### Vapor-liquid equilibrium

To understand the real behavior of the mixtures, experimental vapor-liquid equilibrium (VLE) data have been measured for monovalent alcohol and hexanediamine mixtures. These were binary isobaric VLE measurements of the monovalent alcohols 1-butanol, 1-pentanol and 1-hexanol in combination with hexanediamine. The measurements of each binary VLE have been performed for three different pressures.

In summary, 9 experiments have been performed. These were conducted in a dynamic circulation still (ebulliometer as a standard method well known in the art) with sampling of condensed vapor phase and liquid phase followed by a gas-chromatographic quantification of alcohol and diamine. The pressure in the cell was held constant and boiling temperatures of mixtures with different concentrations were measured.

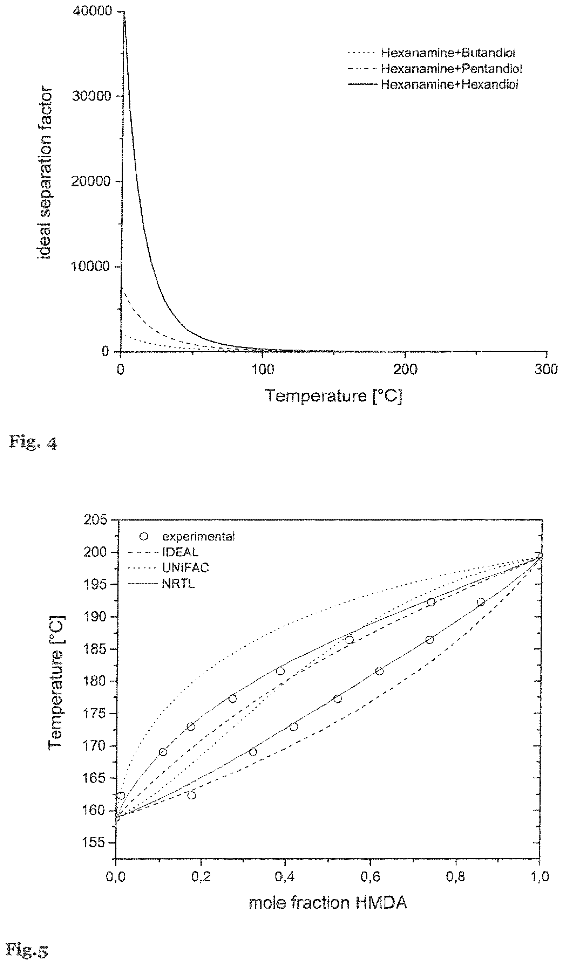

In Fig.5, the measured VLE of 1-hexanol and hexanediamine at 1000 mbar is shown exemplarily. Experimental data is compared to both an ideal VLE determination and an VLE estimation with a predictive thermodynamic model named UNIFAC ("Universal Quasichemical Functional Group Activity Coefficients").

In Fig. 5, points indicate experimental data, solid line is calculation with NRTL model (with regressed parameters), dashed line is an ideal calculation, dotted line is a prediction with UNIFAC estimation method ("Universal Quasichemical Functional Group Activity Coefficients", known in the art). In Fig. 5, an ideal calculation of the phase equilibrium and an UNIFAC estimation is included. It can be clearly seen that the experimental data (data points) deviate from both of these. This makes it obvious that without measurements, the real behavior of the systems could not be predicted properly.

Model parameters of the NRTL ("non-random-two-liquid", known in the art) model for all investigated systems have been regressed to the measured data (solid line in Fig. 5). It can be stated that the model fits nicely to the data. The regressed parameters have been used in all simulation studies from which insights regarding the process have been retrieved. This means that all simulation studies are based on experimental VLE data for several monovalent alcohol+hexanediamine experiments at different pressures.

### Effect of operating pressure on energy demand

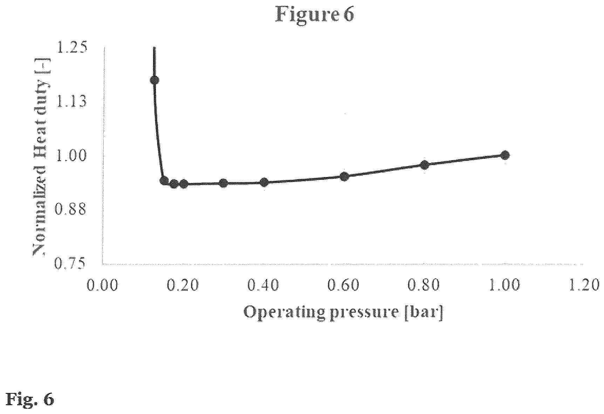

The effect of the distillation column operating pressure on the energy demand of the separation was investigated in more depth using a rigorous process model based on the experimentally regressed NRTL parameters. For the exemplary mixture of 1-hexanol and hexanediamine used before (see Fig. 5), the influence of the operating pressure on the energy demand of separation is depicted in Fig. 6. For the simulations, the composition of the mixture to be separated is representative for the extract phase from the previous liquid-liquid-extraction step and also includes water present due to cross solubility in 1-hexanol as the exemplary solvent. The energy demand for the distillative separation of hexanediamine from 1-hexanol and water shows a significant dependency on the applied column pressure. By decreasing the column pressure from atmospheric pressure to vacuum, the heat duty for the distillation decreased and thus, the separation becomes more energy efficient. For operating pressure between 200 to 400 mbar, an optimum in the applied pressure is found. However, when the pressure is decreased further, due to the thermodynamic behavior of the binary system 1-hexanol / hexanediamine the energy demand for the separation starts to increase significantly. This significant increase is directly related to the non-ideal behavior of the mixture and could be quantified based on the experimental VLE measurements performed for the different pressure levels as shown exemplarily in Fig. 5.

The features disclosed in the foregoing description, the claims as well as the drawing may, both separately and in any combination thereof, be material for realizing the aspects of the disclosure made in the independent claims, in diverse forms thereof.

## Claims

1. Method for separating HMDA from an aqueous solution, comprising the steps:
a) providing an HMDA-containing aqueous solution, wherein said aqueous solution is a fermentation broth,
b) extracting the HMDA-containing aqueous solution with a monovalent alcohol having 4 to 8 carbon atoms,
c) obtaining an HMDA-containing alcoholic phase, and
d) distilling the HMDA-containing alcoholic phase at a pressure of 1-1000 mbar and separating the HMDA from the monovalent alcohol.

2. Method according to claim 1, wherein the aqueous solution comprises at least one compound selected from the group consisting of (i) acetate, (ii) lactate, (iii) glutamate, (iv) ammonium and (v) y-aminobutyric acid.

3. Method according to claim 2, wherein the concentration range of acetate is between 1 and 4,000 mg/L, the concentration range of lactate is between 1 and 400 mg/L, the concentration range of glutamate is between 10 and 1,000 mg/L, the concentration range of ammonium is between 1 and 200 mg/L, or the concentration range of y-aminobutyric acid is between 300 and 4,000 mg/L.

4. Method according to any of the preceding claims, wherein the monovalent alcohol is selected from the group consisting of 1-pentanol, 1-hexanol, 1-heptanol or mixtures thereof.

5. Method according to any of the preceding claims, wherein the HM DA-containing alcoholic phase is distilled in step d) at a pressure of 150-400 mbar.

6. Method according to any of the preceding claims, wherein the HMDA-containing alcoholic phase is distilled in step d) with a single distillation column.

7. Method according to any of the preceding claims, wherein step b) is carried out using an extraction column and/or a mixer-settler, having 3 to 15 theoretical stages.
